(19) Europäisches Patentamt
European Patent Office
Office européen des brevets

(11) Veröffentlichungsnummer: **0 018 509**
**B1**

(12) # EUROPÄISCHE PATENTSCHRIFT

(45) Veröffentlichungstag der Patentschrift: **19.05.82**

(21) Anmeldenummer: **80101842.5**

(22) Anmeldetag: **05.04.80**

**Die Akte enthält technische Angaben, die nach dem Eingang der Anmeldung eingereicht wurden und die nicht in dieser Patentschrift enthalten sind.**

(51) Int. Cl.³: **C 07 D307/68**, C 07 D249/08, C 07 D261/18, C 07 C103/365, C 07 C103/375, A 01 N 43/06, A 01 N 43/64, A 01 N 43/80, A 01 N 37/22// C07D233/56, C07D231/12

(54) **N-Allenyl-acetanilide, Verfahren zu ihrer Herstellung sowie ihre Verwendung als Fungizide.**

(30) Priorität: **25.04.79 DE 2916692**

(43) Veröffentlichungstag der Anmeldung: **12.11.80 (Patentblatt 80/23)**

(45) Bekanntmachung des Hinweises auf die Patenterteilung: **19.05.82 Patentblatt 82/20**

(84) Benannte Vertragsstaaten: **AT BE CH DE FR GB IT LI NL**

(56) Entgegenhaltungen:

**EP - A - 0 005 591**
**US - A - 4 021 224**

**CHEMICAL ABSTRACTS, Band 89, Nr. 9, 28. August 1978, Zusammenfassung Nr. 75356s, Seite 405, Columbus, Ohio, US, T.R. MELIKYAN et al.: « Studies on amines and ammonium compounds. CXXXIX. Intramolecular cyclization of N-allylcinnamamides and N-allylfurancarboxylic acid amides ».**

**TETRAHEDRON LETTERS, Nr. 7, Februar 1979, Pergamon Press, Oxford, GB, L.E. OVERMAN et al.: « The preparation of N-trichloroacetamido-1, 2-dienes », Seiten 599, 600**

(73) Patentinhaber: **BAYER AG**
**Zentralbereich Patente, Marken und Lizenzen**
**D-5090 Leverkusen 1, Bayerwerk (DE)**

(72) Erfinder: **Stetter, Jörg, Dr.**
**Pahlkestrasse 3**
**D-5600 Wuppertal-1 (DE)**
Erfinder: **Lunkenheimer, Winfried, Dr.**
**Bismarckstrasse 29**
**D-5600 Wuppertal-1 (DE)**
Erfinder: **Brandes, Wilhelm, Dr.**
**Eichendorffstrasse 3**
**D-5623 Leichlingen (DE)**

EP 0 018 509 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

N-Allenyl-acetanilid, Verfahren zu ihrer Herstellung sowie ihre Verwendung als Fungizide

Die Erfindung betrifft neue N-Allenyl-acetanilide, ein Verfahren zu ihrer Herstellung und ihre Verwendung als Fungizide.

Es ist bereits bekannt, daß Halogenacetanilide, wie beispielsweise N-Chloracetyl-N-(2,6-dialkylphenyl)-alanin- bzw. -glycin-alkylester mit gutem Erfolg zur Bekämpfung von pilzlichen Pflanzenkrankheiten eingesetzt werden können (vgl. DT-OS 2 350 344 bzw. US-PS 3 780 090). Deren Wirkung ist jedoch, insbesondere bei niedrigen Aufwandmengen und -konzentrationen, insbesondere auch bei der Bekämpfung von Phytophthora-Arten, nicht immer ganz befriedigend.

Es wurden neue N-Allenyl-acetanilide der allgemeinen Formel I

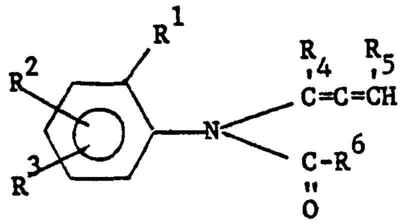

(I)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff oder Alkyl mit 1 bis 4 C-Atomen steht,

$R^5$ für Wasserstoff oder Alkyl mit 1 bis 4 C-Atomen steht,

$R^6$ für Furyl, Tetrahydrofuryl, Thiophenyl, Tetrahydrothiophenyl ; gegebenenfalls durch $C_1$-$C_2$-Alkyl substituiertes Isoxazolyl ; gegebenenfalls durch Cyano oder Thiocyano substituiertes $C_1$-$C_4$-Alkyl, $C_2$-$C_4$-Alkenyl und $C_2$-$C_4$-Alkinyl ; $C_1$-$C_2$-Dihalogenalkyl ; sowie die Gruppierungen $-CH_2-Az$, $-CH_2-OR^7$,

$-CH_2-Sr^7$, $-OR^7$, $-SR^7$, $-CH_2-OSO_2R^7$, $-COOR^7$ und $-CH_2-O-$ 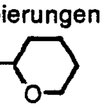

steht, wobei

$R^7$ für gegebenenfalls durch Halogen, Cyano und Thiocyano substituiertes Alkyl mit 1 bis 4 C-Atomen, $C_2$-$C_4$-Alkenyl, $C_2$-$C_4$-Alkinyl und Alkoxyalkyl mit 1 bis 4 C-Atomen steht, und

Az für Pyrazol-1-yl, 1,2,4-Triazol-1-yl und Imidazol-1-yl steht,

gefunden.

Weitherhin wurde gefunden, daß man die neuen N-Allenyl-acetanilide der allgemeinen Formel I erhält, wenn man N-Propargyl-acetanilide der allgemeinen Formel II

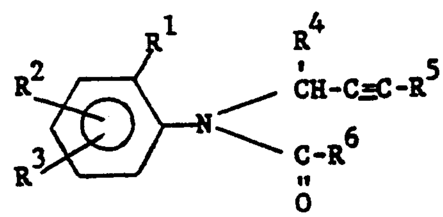

(II)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff oder Alkyl mit 1 bis 4 C-Atomen steht,

$R^5$ für Wasserstoff oder Alkyl mit 1 bis 4 C-Atomen steht,

$R^6$ für Furyl, Tetrahydrofuryl, Thiophenyl, Tetrahydrothiophenyl ; gegebenenfalls durch $C_1$-$C_2$-Alkyl substituiertes Isoxazolyl ; gegebenenfalls durch Cyano oder Thiocyano substituiertes $C_1$-$C_4$-Alkyl, $C_2$-$C_4$-Alkenyl und $C_2$-$C_4$-Alkinyl ; $C_1$-$C_2$-Dihalogenalkyl ; sowie die Gruppierungen $-CH_2-Az$, $-CH_2-OR^7$,

$-CH_2-SR^7$, $-OR^7-SR^7$, $-CH_2-OSO_2R^7$, $-COOR^7$ und $-CH_2-O-$ 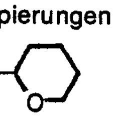

steht, wobei

$R^7$ für gegebenenfalls durch Halogen, Cyano und Thiocyano substituiertes Alkyl mit 1 bis 4 C-Atomen, $C_2$-$C_4$-Alkenyl, $C_2$-$C_4$-Alkinyl und Alkoxyalkyl mit 1 bis 4 C-Atomen steht, und

Az für Pyrazol-1-yl, 1,2,4-Triazol-1-yl und Imidazol-1-yl steht.

in Gegenwart einer Base als Katalysator und gegebenenfalls in Gegenwart eines Verdünnungsmittels umlagert.

Weiterhin wurde gefunden, daß die neuen N-Allenyl-acetanilide der allgemeinen Formel I starke fungizide Eigenschaften aufweisen. Dabei zeigen überraschenderweise die erfindungsgemäßen Verbindungen eine erheblich höhere Wirkung, insbesondere gegen Phytophthora, als die aus dem Stand der Technik bekannten N-Chloracetyl-N-(2,6-dialkylphenyl)-alanin-bzw.-glycin-alkylester.

Die erfindungsgemäßen substituierten N-Allenyl-acetanilide sind durch die allgemeine Formel I definiert. In dieser Formel stehen $R^1$, $R^2$, $R^3$, $R^4$ und $R^5$ für Wasserstoff und geradkettiges oder verzweigtes Alkyl mit 1 bis 4 Kohlenstoffatomen, $R^1$ steht außerdem für Halogen, wie insbesondere Fluor, Chlor oder Brom. $R^6$ steht für Furyl, Tetrahydrofuryl, Thiophenyl, Tetrahydrothiophenyl, für gegebenenfalls durch Methyl oder Ethyl substituiertes Isoxazolyl sowie für gegebenenfalls durch Cyano oder Thiocyano substituiertes Alkyl mit 1 bis 4 und Alkenyl sowie Alkinyl mit jeweils 2 bis 4 Kohlenstoffatomen ; ferner für Dihalogenalkyl mit 1 bis 2 Kohlenstoffatomen, wobei als Halogenatome vorzugsweise Fluor und Chlor genannt seien, sowie für die Gruppierungen $—CH_2—Az$, $CH_2—OR^7$, $—CH_2—SR^7$, $—OR^7$, $—SR^7$, $—CH_2—OSO_2R^7$, $—COOR^7$ und $—CH_2—O—$ 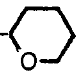

Az steht dabei für Pyrazol-1-yl, 1,2,4-Triazol-1-yl und Imidazol-1-yl. $R^7$ steht für gegebenenfalls durch Halogen, insbesondere Fluor, Chlor und Brom, Cyano und Thiocyano substituiertes Alkyl mit 1 bis 4 und Alkenyl sowie Alkinyl mit jeweils 2 bis 4 Kohlenstoffatomen und für Alkoxyalkyl mit 1 bis 4 Kohlenstoffatomen in jedem Alkylteil.

Ganz besonders bevorzugt sind diejenigen substituierten N-Propargyl-aniline der allgemeinen Formel I, in denen $R^1$, $R^2$ und $R^3$ für Wasserstoff, Methyl, Ethyl, Isopropyl, sek.-Butyl und tert.-Butyl stehen ; $R^1$ außerdem für Chlor oder Brom steht ; $R^4$ und $R^5$ für Wasserstoff, Methyl oder Ethyl stehen ; und $R^6$ für 2-Furyl, 2-Thienyl, 2-Tetrahydrofuryl, Vinyl, 5-Methylisoxazol-3-yl, Methoxymethyl, Ethoxymethyl, Allyloxymethyl, Propargyloxymethyl, Ethoxymethoxymethyl, Methylmercaptomethyl, Methoxy, Ethoxy, Methylmercapto, Methylsulfonyloxymethyl, Methoxycarbonyl, Ethoxycarbonyl, Propargyloxycarbonyl, Dichlormethyl, Pyrazol-1-yl-methyl, Imidazol,-1-yl-methyl, 1,2,4-Triazol-1-yl-methyl und Tetrahydropyran-2-yl-oxy-methyl steht.

Im einzelnen seien außer den bei den Herstellungsbeispielen genannten Verbindungen die folgenden Verbindungen der allgemeinen Formel I genannt :

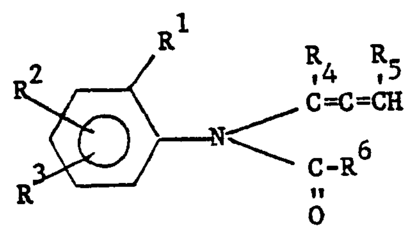

(I)

| $R^1$ | $R^2$ | $R^3$ | $R^4$ | $R^5$ | $R^6$ |
|---|---|---|---|---|---|
| $CH_3$ | 6-$CH_3$ | H | H | H | $-CH_2-$ 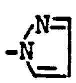 |
| $CH_3$ | 6-$CH_3$ | H | H | H | $-CH_2-$ 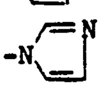 |
| $CH_3$ | 6-$CH_3$ | H | H | H | $-CH_2-O-CH_2-OC_2H_5$ |
| $CH_3$ | 6-$CH_3$ | H | H | H | $-CH_2-O-$ 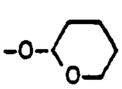 |
| $CH_3$ | 6-$CH_3$ | H | H | H | 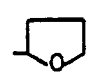 |
| $CH_3$ | 6-$CH_3$ | H | H | H | 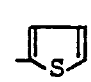 |
| $CH_3$ | 6-$CH_3$ | H | H | H | 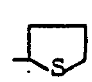 |
| $CH_3$ | 6-$CH_3$ | H | H | H | $-CH_2-S-CH_3$ |

| $R^1$ | $R^2$ | $R^3$ | $R^4$ | $R^5$ | $R^6$ |
|---|---|---|---|---|---|
| $CH_3$ | 6-$CH_3$ | H | H | H | $-CH_2-S-C_2H_5$ |
| $CH_3$ | 6-$CH_3$ | H | H | H | $-CHCl_2$ |
| $CH_3$ | 6-$CH_3$ | H | H | H | $-COOCH_3$ |
| $CH_3$ | 6-$CH_3$ | H | H | H | $-CH_2-O-SO_2CH_3$ |
| $CH_3$ | 6-$CH_3$ | H | H | H | $-CO-O-CH_2-C\equiv CH$ |
| $CH_3$ | 6-$CH_3$ | H | H | H | $-CH=CH_2$ |
| $CH_3$ | 6-$CH_3$ | H | $CH_3$ | H | 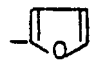 |
| $CH_3$ | 6-$CH_3$ | H | $CH_3$ | H | $-CH_2-O-CH_3$ |
| $CH_3$ | 6-$CH_3$ | H | $CH_3$ | H | $-CH_2-$ 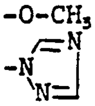 |
| $CH_3$ | 6-$CH_3$ | H | $CH_3$ | H | $-CH_2-$ 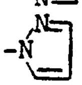 |
| $CH_3$ | 6-$CH_3$ | H | $CH_3$ | H | $-CH_2-$ 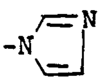 |
| $CH_3$ | 6-$CH_3$ | H | $CH_3$ | H | $-CH_2-OC_2H_5$ |
| $CH_3$ | 6-$CH_3$ | H | $CH_3$ | H | $-CH_2-O-CH_2-OC_2H_5$ |
| $CH_3$ | 6-$CH_3$ | H | $CH_3$ | H | $-CH_2-O-$ 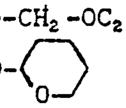 |
| $CH_3$ | 6-$CH_3$ | H | $CH_3$ | H | 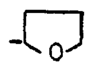 |
| $CH_3$ | 6-$CH_3$ | H | $CH_3$ | H | 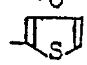 |
| $CH_3$ | 6-$CH_3$ | H | $CH_3$ | H | 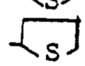 |
| $CH_3$ | 6-$CH_3$ | H | $CH_3$ | H | $-CH_2-S-CH_3$ |
| $CH_3$ | 6-$CH_3$ | H | $CH_3$ | H | $-CH_2-S-C_2H_5$ |
| $CH_3$ | 6-$CH_3$ | H | $CH_3$ | H | $-CHCl_2$ |
| $CH_3$ | 6-$CH_3$ | H | $CH_3$ | H | $-COOCH_3$ |
| $CH_3$ | 6-$CH_3$ | H | $CH_3$ | H | $-CH_2-O-SO_2CH_3$ |
| $CH_3$ | 6-$CH_3$ | H | $CH_3$ | H | $-CO-OCH_2-C\equiv CH$ |
| $CH_3$ | 6-$CH_3$ | H | $CH_3$ | H | $-CH=CH_2$ |
| $CH_3$ | 6-$CH_3$ | H | H | $CH_3$ | 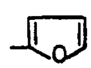 |
| $CH_3$ | 6-$CH_3$ | H | H | $CH_3$ | $-CH_2-OCH_3$ |
| $CH_3$ | 6-$CH_3$ | H | H | $CH_3$ | $-CH_2-$ 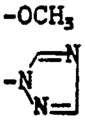 |
| $CH_3$ | 6-$CH_3$ | H | H | $CH_3$ | $-CH_2-$ 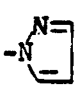 |

| $R^1$ | $R^2$ | $R^3$ | $R^4$ | $R^5$ | $R^6$ |
|---|---|---|---|---|---|
| $CH_3$ | 6-$CH_3$ | H | H | $CH_3$ | $-CH_2-$ 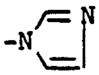 |
| $CH_3$ | 6-$CH_3$ | H | H | $CH_3$ | $-CH_2-OC_2H_5$ |
| $CH_3$ | 6-$CH_3$ | H | H | $CH_3$ | $-CH_2-O-CH_2-OC_2H_5$ |
| $CH_3$ | 6-$CH_3$ | H | H | $CH_3$ | $-CH_2-O-$ 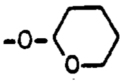 |
| $CH_3$ | 6-$CH_3$ | H | H | $CH_3$ | 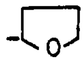 |
| $CH_3$ | 6-$CH_3$ | H | H | $CH_3$ | 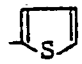 |
| $CH_3$ | 6-$CH_3$ | H | H | $CH_3$ | 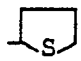 |
| $CH_3$ | 6-$CH_3$ | H | H | $CH_3$ | $-CH_2-SCH_3$ |
| $CH_3$ | 6-$CH_3$ | H | H | $CH_3$ | $-CH_2-SC_2H_5$ |
| $CH_3$ | 6-$CH_3$ | H | H | $CH_3$ | $-CHCl_2$ |
| $CH_3$ | 6-$CH_3$ | H | H | $CH_3$ | $-COOCH_3$ |
| $CH_3$ | 6-$CH_3$ | H | H | $CH_3$ | $-CH_2-O-SO_2CH_3$ |
| $CH_3$ | 6-$CH_3$ | H | H | $CH_3$ | $-CO-O-CH_2-C\equiv CH$ |
| $CH_3$ | 6-$CH_3$ | H | H | $CH_3$ | $-CH=CH_2$ |
| $CH_3$ | 6-$CH_3$ | H | $CH_3$ | $CH_3$ | 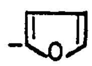 |
| $CH_3$ | 6-$CH_3$ | H | $CH_3$ | $CH_3$ | $-CH_2-OCH_3$ |
| $CH_3$ | 6-$CH_3$ | H | $CH_3$ | $CH_3$ | $-CH_2-$ 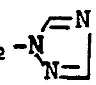 |
| $CH_3$ | 5-$CH_3$ | H | $CH_3$ | $CH_3$ | $-CH_2-$ 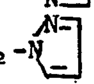 |
| $CH_3$ | 6-$CH_3$ | H | $CH_3$ | $CH_3$ | $-CH_2-$ 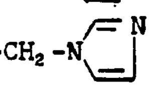 |
| $CH_3$ | 6-$CH_3$ | H | $CH_3$ | $CH_3$ | $-CH_2-OC_2H_5$ |
| $CH_3$ | 6-$CH_3$ | H | $CH_3$ | $CH_3$ | $-CH_2-O-CH_2-OC_2H_5$ |
| $CH_3$ | 6-$CH_3$ | H | $CH_3$ | $CH_3$ | $-CH_2-O-$ 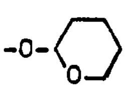 |
| $CH_3$ | 6-$CH_3$ | H | $CH_3$ | $CH_3$ | 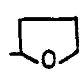 |
| $CH_3$ | 6-$CH_3$ | H | $CH_3$ | $CH_3$ | 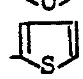 |
| $CH_3$ | 6-$CH_3$ | H | $CH_3$ | $CH_3$ | 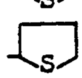 |
| $CH_3$ | 6-$CH_3$ | H | $CH_3$ | $CH_3$ | $-CH_2-SCH_3$ |
| $CH_3$ | 6-$CH_3$ | H | $CH_3$ | $CH_3$ | $-CH_2-SC_2H_5$ |
| $CH_3$ | 6-$CH_3$ | H | $CH_3$ | $CH_3$ | $-CHCl_2$ |
| $CH_3$ | 6-$CH_3$ | H | $CH_3$ | $CH_3$ | $-COOCH_3$ |

| $R^1$ | $R^2$ | $R^3$ | $R^4$ | $R^5$ | $R^6$ |
|---|---|---|---|---|---|
| $CH_3$ | 6-$CH_3$ | H | $CH_3$ | $CH_3$ | $-CH_2-O-SO_2CH_3$ |
| $CH_3$ | 6-$CH_3$ | H | $CH_3$ | $CH_3$ | $-CO-O-CH_2-C{\equiv}CH$ |
| $CH_3$ | 6-$CH_3$ | H | $CH_3$ | $CH_3$ | $-CH{=}CH_2$ |
| $CH_3$ | 6-$C_2H_5$ | H | H | H | 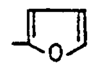 |
| $CH_3$ | 6-$C_2H_5$ | H | H | H | $-CH_2-OCH_3$ |
| $CH_3$ | 6-$C_2H_5$ | H | H | H | $-CH_2-$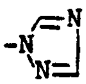 |
| $CH_3$ | 6-$C_2H_5$ | H | H | H | $-CH_2-$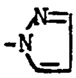 |
| $CH_3$ | 6-$C_2H_5$ | H | H | H | $-CH_2-O-C_2H_5$ |
| $CH_3$ | 6-$C_2H_5$ | H | H | H | $-CH_2-O-SO_2CH_3$ |
| $CH_3$ | 6-$C_2H_5$ | H | H | H | $-CHCl_2$ |
| $CH_3$ | 6-$C_2H_5$ | H | H | H | $-COOCH_3$ |
| $CH_3$ | 6-$C_2H_5$ | H | H | H | 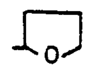 |
| $CH_3$ | 6-$C_2H_5$ | H | H | H | $-CH_2-SCH_3$ |
| $CH_3$ | 6-$C_2H_5$ | H | H | H | $-CH_2-O-$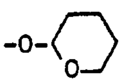 |
| $CH_3$ | 6-$C_2H_5$ | H | H | H | $-CH{=}CH_2$ |
| $CH_3$ | 6-$C_2H_5$ | H | $CH_3$ | H | 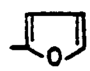 |
| $CH_3$ | 6-$C_2H_5$ | H | $CH_3$ | H | $-CH_2-OCH_3$ |
| $CH_3$ | 6-$C_2H_5$ | H | $CH_3$ | H | $-CH_2-$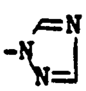 |
| $CH_3$ | 6-$C_2H_5$ | H | $CH_3$ | H | $-CH_2-$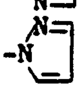 |
| $CH_3$ | 6-$C_2H_5$ | H | $CH_3$ | H | $-CH_2-O-C_2H_5$ |
| $CH_3$ | 6-$C_2H_5$ | H | $CH_3$ | H | $-CH_2-O-SO_2CH_3$ |
| $CH_3$ | 6-$C_2H_5$ | H | $CH_3$ | H | $-CHCl_2$ |
| $CH_3$ | 6-$C_2H_5$ | H | $CH_3$ | H | $-COOCH_3$ |
| $CH_3$ | 6-$C_2H_5$ | H | $CH_3$ | H | 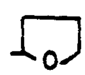 |
| $CH_3$ | 6-$C_2H_5$ | H | $CH_3$ | H | $-CH_2-SCH_3$ |
| $CH_3$ | 6-$C_2H_5$ | H | $CH_3$ | H | $-CH_2-O-$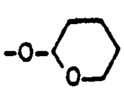 |
| $CH_3$ | 6-$C_2H_5$ | H | $CH_3$ | H | $-CH{=}CH_2$ |

| $R^1$ | $R^2$ | $R^3$ | $R^4$ | $R^5$ | $R^6$ |
|---|---|---|---|---|---|
| $CH_3$ | $6-C_2H_5$ | H | H | $CH_3$ | 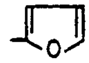 |
| $CH_3$ | $6-C_2H_5$ | H | H | $CH_3$ | $-CH_2-OCH_3$ |
| $CH_3$ | $6-C_2H_5$ | H | H | $CH_3$ | $-CH_2-$ 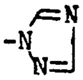 |
| $CH_3$ | $6-C_2H_5$ | H | H | $CH_3$ | $-CH_2-$ 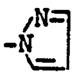 |
| $CH_3$ | $6-C_2H_5$ | H | H | $CH_3$ | $-CH_2-O-C_2H_5$ |
| $CH_3$ | $6-C_2H_5$ | H | H | $CH_3$ | $-CH_2-O-SO_2CH_3$ |
| $CH_3$ | $6-C_2H_5$ | H | H | $CH_3$ | $-CHCl_2$ |
| $CH_3$ | $6-C_2H_5$ | H | H | $CH_3$ | $-COOCH_3$ |
| $CH_3$ | $6-C_2H_5$ | H | H | $CH_3$ | 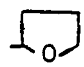 |
| $CH_3$ | $6-C_2H_5$ | H | H | $CH_3$ | $-CH_2-SCH_3$ |
| $CH_3$ | $6-C_2H_5$ | H | H | $CH_3$ | $-CH_2-O-$ 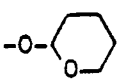 |
| $CH_3$ | $6-C_2H_5$ | H | H | $CH_3$ | $-CH=CH_2$ |
| $C_2H_5$ | $6-C_2H_5$ | H | H | H | 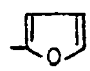 |
| $C_2H_5$ | $6-C_2H_5$ | H | H | H | $-CH_2-OCH_3$ |
| $C_2H_5$ | $6-C_2H_5$ | H | H | H | $-CH_2-$ 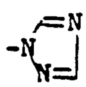 |
| $C_2H_5$ | $6-C_2H_5$ | H | H | H | $-CH_2-$ 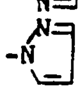 |
| $C_2H_5$ | $6-C_2H_5$ | H | H | H | $-CH_2-O-C_2H_5$ |
| $C_2H_5$ | $6-C_2H_5$ | H | H | H | $-CH_2-O-SO_2CH_3$ |
| $C_2H_5$ | $6-C_2H_5$ | H | H | H | $-CHCl_2$ |
| $C_2H_5$ | $6-C_2H_5$ | H | H | H | $-COOCH_3$ |
| $C_2H_5$ | $6-C_2H_5$ | H | H | H | 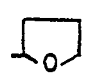 |
| $C_2H_5$ | $6-C_2H_5$ | H | H | H | $-CH_2-SCH_3$ |
| $C_2H_5$ | $6-C_2H_5$ | H | H | H | $-CH_2-O-$ 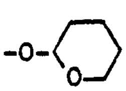 |
| $C_2H_5$ | $6-C_2H_5$ | H | H | H | $-CH=CH_2$ |
| $C_2H_5$ | $6-C_2H_5$ | H | $CH_3$ | H | 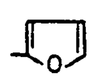 |
| $C_2H_5$ | $6-C_2H_5$ | H | $CH_3$ | H | $-CH_2-OCH_3$ |
| $C_2H_5$ | $6-C_2H_5$ | H | $CH_3$ | H | $-CH_2-$ 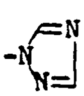 |

| $R^1$ | $R^2$ | $R^3$ | $R^4$ | $R^5$ | $R^6$ |
|---|---|---|---|---|---|
| $C_2H_5$ | $6-C_2H_5$ | H | $CH_3$ | H | $-CH_2-$ 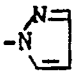 |
| $C_2H_5$ | $6-C_2H_5$ | H | $CH_3$ | H | $-CH_2-O-C_2H_5$ |
| $C_2H_5$ | $6-C_2H_5$ | H | $CH_3$ | H | $-CH_2-O-SO_2CH_3$ |
| $C_2H_5$ | $6-C_2H_5$ | H | $CH_3$ | H | $-CHCl_2$ |
| $C_2H_5$ | $6-C_2H_5$ | H | $CH_3$ | H | $-COOCH_3$ |
| $C_2H_5$ | $6-C_2H_5$ | H | $CH_3$ | H | 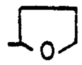 |
| $C_2H_5$ | $6-C_2H_5$ | H | $CH_3$ | H | $-CH_2-SCH_3$ |
| $C_2H_5$ | $6-C_2H_5$ | H | $CH_3$ | H | $-CH_2-O-$ 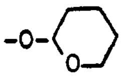 |
| $C_2H_5$ | $6-C_2H_5$ | H | $CH_3$ | H | $-CH=CH_2$ |
| $C_2H_5$ | $6-C_2H_5$ | H | H | $CH_3$ | 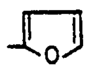 |
| $C_2H_5$ | $6-C_2H_5$ | H | H | $CH_3$ | $-CH_2-OCH_3$ |
| $C_2H_5$ | $6-C_2H_5$ | H | H | $CH_3$ | $-CH_2-$ 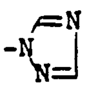 |
| $C_2H_5$ | $6-C_2H_5$ | H | H | $CH_3$ | $-CH_2-$ 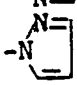 |
| $C_2H_5$ | $6-C_2H_5$ | H | H | $CH_3$ | $-CH_2-O-C_2H_5$ |
| $C_2H_5$ | $6-C_2H_5$ | H | H | $CH_3$ | $-CH_2-O-SO_2CH_3$ |
| $C_2H_5$ | $6-C_2H_5$ | H | H | $CH_3$ | $-CHCl_2$ |
| $C_2H_5$ | $6-C_2H_5$ | H | H | $CH_3$ | $-COOCH_3$ |
| $C_2H_5$ | $6-C_2H_5$ | H | H | $CH_3$ | 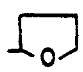 |
| $C_2H_5$ | $6-C_2H_5$ | H | H | $CH_3$ | $-CH_2-SCH_3$ |
| $C_2H_5$ | $6-C_2H_5$ | H | H | $CH_3$ | $-CH_2-O-$ 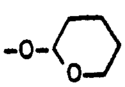 |
| $C_2H_5$ | $6-C_2H_5$ | H | H | $CH_3$ | $-CH=CH_2$ |
| $C(CH_3)_3$ | H | H | H | H | 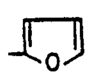 |
| $C(CH_3)_3$ | H | H | H | H | $-CH_2-OCH_3$ |
| $C(CH_3)_3$ | H | H | H | H | $-CH_2-$ 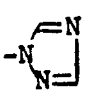 |
| $C(CH_3)_3$ | H | H | H | H | $-CH_2-$ 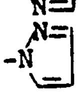 |
| $C(CH_3)_3$ | H | H | H | H | $-CH_2-O-C_2H_5$ |
| $C(CH_3)_3$ | H | H | H | H | $-CH_2-O-SO_2CH_3$ |

| $R^1$ | $R^2$ | $R^3$ | $R^4$ | $R^5$ | $R^6$ |
|---|---|---|---|---|---|
| $C(CH_3)_3$ | H | H | H | H | $-CHCl_2$ |
| $C(CH_3)_3$ | H | H | H | H | $-COOCH_3$ |
| $C(CH_3)_3$ | H | H | H | H | 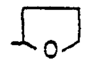 |
| $C(CH_3)_3$ | H | H | H | H | $-CH_2-SCH_3$ |
| $C(CH_3)_3$ | H | H | H | H | $-CH_2-O-$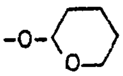 |
| $C(CH_3)_3$ | H | H | H | H | $-CH=CH_2$ |
| Cl | $6-CH_3$ | H | H | H | 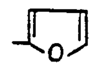 |
| Cl | $6-CH_3$ | H | H | H | $-CH_2-OCH_3$ |
| Cl | $6-CH_3$ | H | H | H | $-CH_2-$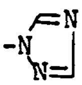 |
| Cl | $6-CH_3$ | H | H | H | $-CH_2-$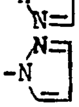 |
| Cl | $6-CH_3$ | H | H | H | $-CH_2-O-C_2H_5$ |
| Cl | $6-CH_3$ | H | H | H | $-CH_2-O-SO_2CH_3$ |
| Cl | $6-CH_3$ | H | H | H | $-CHCl_2$ |
| Cl | $6-CH_3$ | H | H | H | $-COOCH_3$ |
| Cl | $6-CH_3$ | H | H | H | 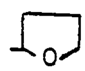 |
| Cl | $6-CH_3$ | H | H | H | $-CH_2-SCH_3$ |
| Cl | $6-CH_3$ | H | H | H | $-CH_2-O-$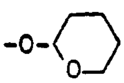 |
| Cl | $6-CH_3$ | H | H | H | $-CH=CH_2$ |
| $CH_3$ | $3-CH_3$ | $6-CH_3$ | H | H | 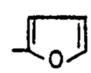 |
| $CH_3$ | $3-CH_3$ | $6-CH_3$ | H | H | $-CH_2-OCH_3$ |
| $CH_3$ | $3-CH_3$ | $6-CH_3$ | H | H | $-CH_2-$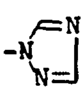 |
| $CH_3$ | $3-CH_3$ | $6-CH_3$ | H | H | $-CH_2-$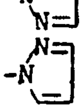 |
| $CH_3$ | $3-CH_3$ | $6-CH_3$ | H | H | $-CH_2-O-C_2H_5$ |
| $CH_3$ | $3-CH_3$ | $6-CH_3$ | H | H | $-CH_2-O-SO_2CH_3$ |
| $CH_3$ | $3-CH_3$ | $6-CH_3$ | H | H | $-CHCl_2$ |
| $CH_3$ | $3-CH_3$ | $6-CH_3$ | H | H | $-COOCH_3$ |
| $CH_3$ | $3-CH_3$ | $6-CH_3$ | H | H | 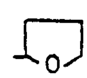 |
| $CH_3$ | $3-CH_3$ | $6-CH_3$ | H | H | $-CH_2-SCH_3$ |

| $R^1$ | $R^2$ | $R^3$ | $R^4$ | $R^5$ | $R^6$ |
|---|---|---|---|---|---|
| $CH_3$ | 3-$CH_3$ | 6-$CH_3$ | H | H | 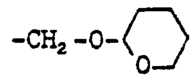 |
| $CH_3$ | 3-$CH_3$ | 6-$CH_3$ | H | H | $-CH=CH_2$ |

Verwendet man beispielsweise 2,6-Dimethyl-N-(2-furoyl)-N-propargyl-anilin als Ausgangsstoff und Kalkum-tert.-butylat als Base, so kann der Reaktionsablauf durch das folgende Formelschema wiedergegeben werden:

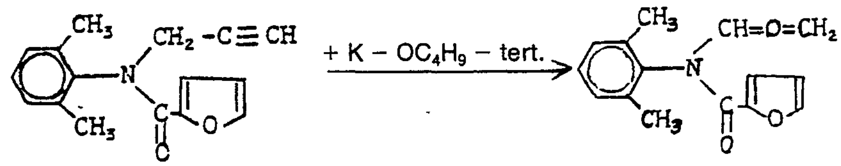


Die bei der Durchführung des erfindungsgemäßen Verfahrens als Ausgangsstoffe benötigten N-Propargyl-acetanilide sind durch die allgemeine Formel II definiert. In dieser Formel stehen $R^1$, $R^2$, $R^3$, $R^4$, $R^5$ und $R^6$ vorzugsweise für diejenigen Reste, die bereits im Zusammenshang mit der Beschreibung der erfindungsgemäßen Stoffe der allgemeinen Formel I vorzugsweise für diese Substituenten genannt wurden.

Die N-Propargyl-acetylanilide der allgemeinen Formel II sind zum Teil aus der EP-A-0005591 bekannt. Die N-Propargyl-acetanilide der allgemeinen Formel II können nach dem in der EP-A-0010673 beschriebenen Verfahren erhalten werden, indem man z.B.

a) Propargyl-aniline der allgemeinen Formel III

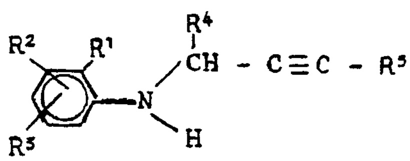
(III)

in welcher

$R^1$ bis $R^5$ die oben angegebene Bedeutung haben,

mit Säurechloriden oder -bromiden bzw. -anhydriden der allgemeinen Formeln IVa bzw. IVb

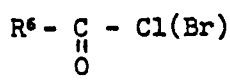
(IVa)

bzw.

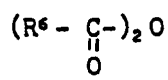
(IVb)

in welchen

$R^6$ die oben angegebene Bedeutung hat,

gegebenenfalls in Gegenwart eines inerten organischen Lösungsmittels, wie beispielsweise Tetrahydrofuran, und in Gegenwart eines Säurebindemittels, wie beispielsweise Triethylamin oder Pyridin, bei Temperaturen zwischen 20 und 100 °C umsetzt, oder

b) Anilide der allgemeinen Formel V

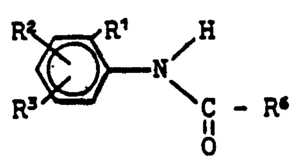
(V)

in welcher
$R^1$, $R^2$, $R^3$ und $R^6$ die oben angegebene Bedeutung haben,
mit Propargylhalogeniden der allgemeinen Formel VI

$$\underset{\underset{R^4}{|}}{\text{Hal} - \text{CH}} - C \equiv C - R^5 \qquad \text{(VI)}$$

in welcher
$R^4$ und $R^5$ die oben angegebene Bedeutung haben und
Hal für Chlor oder Brom steht,
vorzugsweise in einem wässrig-organischen Zweiphasensystem, wie beispielsweise wässrige Natron- oder Kalilauge/Toluol oder Methylenchlorid, gegebenenfalls unter Zusatz von 0,1-1 Mol eines Phasen-Transfer-Katalysators, wie beispielsweise Triethyl-benzyl-ammonium-chlorid, bei Temperaturen zwischen −20 und + 80 °C umsetzt.

Die N-Propargyl-aniline der allgemeinen Formel III sind teilweise bekannt (vgl. die US-PS 3 535 377 und 4 001 325), bzw. können sie nach bekannten Verfahren erhalten werden, indem man z.B. entsprechende Aniline mit Propargylhalogeniden der allgemeinen Formel VI oder den entsprechenden Propargylsufonaten, wie beispielsweise Mesylaten oder Tosylaten, in Gegenwart eines Säurebinders, wie z.B. Natrium- oder Kalium-carbonat, gegebenenfalls in Gegenwart eines inerten organischen Lösungsmittels, wie beispielsweise Ethanol, bei Temperaturen zwischen 20 und 150 °C umsetzt, wobei vorzugsweise auch ein Ueberschuß an Anilin eingesetzt werden kann.

N-Propargyl-aniline der allgemeinen Formel III, bei denen $R^4$ für Methyl steht, können auch durch Umsetzung der entsprechenden Aniline mit Acetylen in Gegenwart von Kupferacetylid unter Druck erhalten werden [vgl. hierzu Liebigs Ann. Chem. *596,* 1 (1955)].

Die Anilide der allgemeinen Formel V können in allgemein bekannter Art und Weise erhalten werden, indem man entsprechende Aniline mit einem Säurechlorid oder -bromid bzw. -anhydrid der allgemeinen Formeln IVa und IVb gemäß den Bedingungen des Verfahrens (a) in Gegenwart eines inerten organischen Lösungsmittels wie beispielsweise Toluol oder Methylenchlorid, gegebenefalls in Gegenwart eines Säurebindemittels, wie z.B. Kaliumcarbonat oder Triethylamin, oder in Gegenwart eines Katalysators, wie z.B. Dimethylformamid, bei Temperaturen zwischen 0 und 100 °C umsetzt.

Die Säurechloride und -bromide bzw. -anhydride der allgemeinen Formeln IVa bzw. IVb sowie die Propargylhalogenide der allgemeinen Formel VI sind allgemeinen bekannte Verbindungen der organischen Chemie.

Als Beispiele für die als Ausgangs stoffe für das erfindungsgemäße Verfahren zu verwendenden N-Propargyl-acetanilide der allgemeinen Formel II seien genannt :

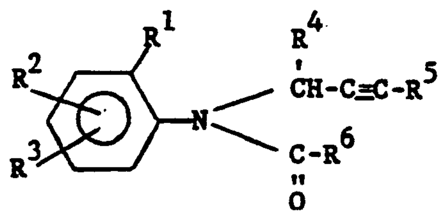

(II)

| $R^1$ | $R^2$ | $R^3$ | $R^4$ | $R^5$ | $R^6$ | Fp(°C) |
|---|---|---|---|---|---|---|
| $CH_3$ | 6-$CH_3$ | H | H | H | 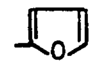 | 109-12 |
| $CH_3$ | 6-$CH_3$ | H | H | H | $-CH_2-O-CH_3$ | 49-51 |
| $C_2H_5$ | 6-$C_2H_5$ | H | H | H | $-CH_2-$ 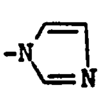 | 129-31 |
| $CH_3$ | 6-$C_2H_5$ | H | H | H | $-CH_2-$ 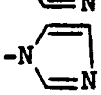 | 129-31 |
| $CH_3$ | 6-$CH_3$ | H | H | $CH_3$ | 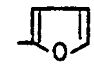 | 110-12 |
| $CH_3$ | 6-$CH_3$ | H | H | H | $-CH_2-$ 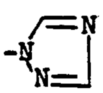 | 120-21 |

| $R^1$ | $R^2$ | $R^3$ | $R^4$ | $R^5$ | $R^6$ | Fp(°C) |
|---|---|---|---|---|---|---|
| $CH_3$ | $6-CH_3$ | H | H | H | $-CH_2-$ 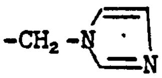 | 137-40 |
| $CH_3$ | $6-C_2H_5$ | H | H | H | 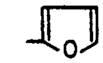 | 88 |
| $CH_3$ | $6-CH_3$ | H | H | $CH_3$ | $-CH_2OCH_3$ | $n_D^{20}$ :1,5362<br>kp:125/C,1mbar |
| $C_2H_5$ | $6-C_2H_5$ | H | H | H | $-CH_2OCH_3$ | 74 |
| $CH_3$ | $6-CH_3$ | H | H | H | $-CHCl_2$ | 114-15 |
| $C_2H_5$ | $6-CH_3$ | H | H | H | 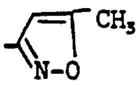 | 98-99 |
| Cl | $6-CH_3$ | H | H | H | $-CH_2OCH_3$ | 80 |
| $CH_3$ | $6-CH_3$ | H | H | H | $-CH_2-$ 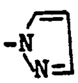 | 93-94 |

Als Verdünnungsmittel kommen für die erfindungsgemäße Umsetzung vorzugsweise inerte organische Lösungsmittel infrage. Hierzu gehören vorzugsweise Ether, wie Tetrahydrofuran oder Dioxan ; Alkohole, wie Methanol, Ethanol oder tert.-Butanol ; sowie Dimethylsulfoxid.

Das erfindungsgemäße Verfahren wird in Gegenwart einer Base als Katalysator durchgeführt. Hierzu werden vorzugsweise Alkalihydroxide oder Alkalicarbonate, wie beispielsweise Natrium- oder Kaliumhydroxid bzw. -carbonat, und insbesondere Alkalialkoholate, wie beispielsweise Natriummethylat, Natriumethylat oder Kalium-tert.-butylat.

Die Reaktionstemperaturen können bei der Durchführung des erfindungsgemäßen Verfahrens in einem größeren Bereich variiert werden. Im allgemeinen arbeitet man zwischen 0 und 120 °C, vorzugsweise zwischen 20 und 80 °C.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Tomate und Kartoffel (Phytophthora infestans) eingesetzt werden. Besonders hervorzuheben ist, daß die erfindungsgemäßen Wirkstoffe nicht nur eine protektive, sondern auch eine kurativ/erdikative Wirkung entfalten. Außerdem besitzen sie systemische Eigenschaften. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoffe-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage : Aromaten, wie Xylol, Toluol, oder Alkylnaphthaline, chlorierte Aromaten oder Chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorrillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsufonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Asol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Staatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,001 bis 0,02 %, am Wirkungsort erforderlich.

In den nachfolgenden Beispielen werden die nachstehend angegebenen Verbindungen als Vergleichssubstanzen eingesetzt:

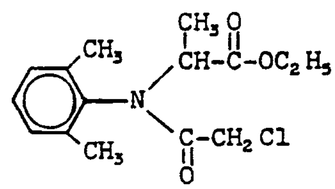

(A)

N-Chloracetyl-N-(2,6-xylyl)-alaninmethylester

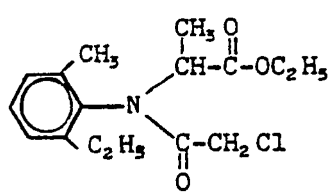

(B)

N-Chloracetyl-N-(2-ethyl-6-methylphenyl)-alaninmethylester

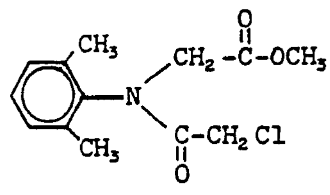

(C)

N-Chloracetyl-N-(2,6-xylyl)-glycinmethylester

Beispiel A

Phytophthora-Test (Tomaten)/Protektiv

Lösungsmittel : 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubbättern bis zur Tropfnässe. Die Pflanzen verbleiben 24 Stunden bei 20 °C und einer relativen Luftfeuchtigkeit von 70 % im Gewächshaus. Anschließend werden die Tomatenpflanzen mit einer wässrigen Sporensuspension von Phytophthora infestans inokuliert. Die Pflanzen werden in eine Feuchtkammer mit einer 100 %igen Luftfeuchtigkeit und einer Temperatur von 18 bis 20 °C gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

In diesem Test zeigen z.B. folgende Verbindungen eine sehr gute Wirkung, die derjenigen der aus dem Stand der Technik bekannten Verbindungen (B) und (C) deutlich überlegen ist :

Verbindungen gemäß Herstellungsbeispielen 1, 2 und 5.

Beispiel B

Phytophthora-Test (Tomaten)/Systemisch

Lösungsmittel : 4,7 Gewichsteile Aceton
Dispergiermittel : 0,3 Gewichsteile Alkyl-aryl-polyglykolether
Wasser : 95,0 Gewichtsteile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden mit 10 ml der Gießflüssigkeit in der angegebenen Wirkstoffkonzentration, bezogen auf 100 ml Erde, gegossen.

Die so behandelten Pflanzen werden nach der Behandlung mit einer wässrigen Sporensuspension von Phytophthora infestans inokuliert. Die Pflanzen werden in eine Feuchtkammer mit einer Luftfeuchtigkeit von 100 % und einer Temperatur von 18 bis 20 °C gebracht. Nach 5 Tagen wird der Befall der Tomatenpflanzen bestimmt. Die so erhaltenen Boniturwerte werden auf Prozent Befall umgerechnet. 0 % bedeutet keinen Befall, 100 % bedeutet, daß die Pflanzen vollständig befallen sind.

In diesem Test zeigen z.B. folgende Verbindungen eine sehr gute Wirkung, die derjenigen der aus dem Stand der Technik bekannten Verbindungen (A) und (B) deutlich überlegen ist :

Verbindungen gemäß Herstellungsbeispiel 1.

Herstellungsbeispiele

Beispiel 1

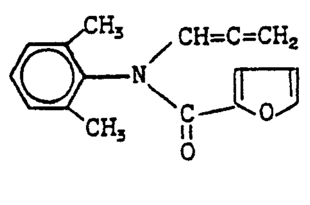

50 g (0,2 Mol) 2,6-Dimethyl-N-(2-furoyl)-N-propargylanilin werden in 200 ml wasserfreiem Tetrahydrofuran gelöst und mit 150 mg Kalium-tert.-butylat versetzt. Dabei steigt unter Verfärbung die Temperatur der Lösung auf ca. 50 °C an. Man läßt zwei Stunden stehen und gießt dann das Reaktionsgemisch auf 500 ml Wasser. Das ausgefallene kristalline Produkt wird abgesaugt, getrocknet und aus Essigester/Petrolether umkristallisiert. Man erhält 24 g (48 % der Theorie) 2,6-Dimethyl-N-allenyl-N-(2-furoyl)-anilin vom Schmelzpunkt 138-41 °C. (Das $^1$H-NMR-Spektrum zeigt die vollständige Isomerisierung an).

Herstellung des Ausgangsproduktes

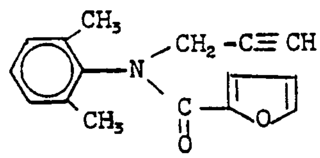

15,9 g (0,1 Mol) 2,6-Dimethyl-N-propargyl-anilin und 8 g (0,1 Mol) Pyridin werden in 100 ml Tetrahydrofuran zum Sieden erhitzt und vorsichtig mit 13 g (0,1 Mol) Furan-2-carbonsäurechlorid versetzt. Man läßt 15 Minuten unter Rückfluß rühren und engt dann die Reaktionsmischung durch Abdestillieren des Lösungsmittels im Vakuum ein. Der Rückstand wird mit Methylenchlorid aufgenommen und mit Wasser gewaschen. Man trennt die organische Phase ab, trocknet über Natriumsulfat und engt ein. Der Rückstand kristallisiert nach dem Verreiben mit Petrolether. Man erhält 22,5 g (89 % der Theorie) 2,6-Dimethyl-N-(2-furoyl)-N-propargylanilin vom Schmelzpunkt 109-112 °C.

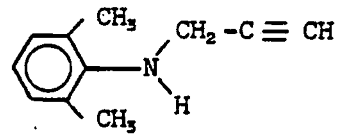

Die Herstellung des 2,6-Dimethyl-N-propargyl-anilins erfolgt durch Umsetzung von 2,6-Dimethylanilin mit Propargylbromid entsprechend den Literaturangaben (vgl. US-PS 4 001 325).

In analoger Weise werden die folgenden Verbindungen der allgemeinen Formel I

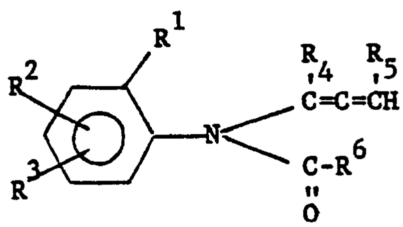

(I)

erhalten :

| Bsp. Nr. | $R^1$ | $R^2$ | $R^3$ | $R^4$ | $R^5$ | $R^6$ | Fp(°C) |
|---|---|---|---|---|---|---|---|
| 2 | $CH_3$ | 6-$CH_3$ | H | H | H | $-CH_2OCH_3$ | 97-99 |
| 3 | $CH_3$ | 6-$CH_3$ | H | H | H | 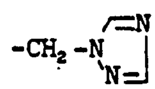 | 117-19 |
| 4 | $CH_3$ | 6-$CH_3$ | H | H | H | 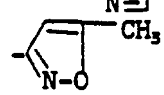 | 98-100 |
| 5 | Cl | 6-$CH_3$ | H | H | H | $-CH_2OCH_3$ | 95-100 |
| 6 | $CH_3$ | 6-$CH_3$ | H | H | H | $-CH_2OC_2H_5$ | 59-60 |

**Ansprüche**

1. N-Allenyl-acetanilide der allgemeinen Formel I

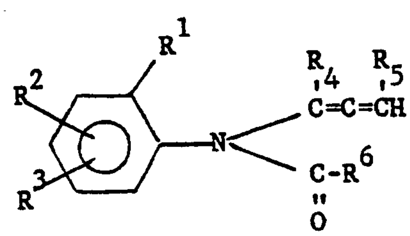

(I)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff oder Alkyl mit 1 bis 4 C-Atomen steht,

$R^5$ für Wasserstoff oder Alkyl mit 1 bis 4 C-Atomen steht,

$R^6$ für Furyl, Tetrahydrofuryl, Thiophenyl, Tetrahydrothiophenyl ; gegebenenfalls durch $C_1$-$C_2$-Alkyl substituiertes Isoxazolyl ; gegebenenfalls durch Cyano oder Thiocyano substituiertes $C_1$-$C_4$-Alkyl, $C_2$-$C_4$-Alkenyl und $C_2$-$C_4$-Alkinyl ; $C_1$-$C_2$-Dihalogenalkyl ; sowie die Gruppierungen $—CH_2—Az$, $—CH_2—OR^7$, $—CH_2—SR^7$, $—OR^7—SR^7$, $—CH_2—OSO_2R^7$, $—COOR^7$ und $—CH_2—O—$ 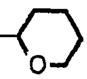

steht, wobei

$R^7$ für gegebenenfalls durch Halogen, Cyano und Thiocyano substituiertes Alkyl mit 1 bis 4 C-Atomen, $C_2$-$C_4$-Alkenyl, $C_2$-$C_4$-Alkinyl und Alkoxyalkyl mit 1 bis 4 C-Atomen steht, und

Az für Pyrazol-1-yl, 1,2,4-Triazol-1-yl und Imidazol-1-yl steht.

2. Verfahren zur Herstellung von N-Allenyl-acetaniliden der allgemeinen Formel I

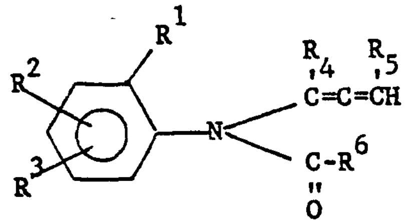

(I)

in welcher

$R^1$ für Wasserstoff, Alkyl mit 1 bis 4 C-Atomen oder Halogen steht,

$R^2$ für Wasserstoff oder Alkyl mit 1 bis 4 C-Atomen steht,

$R^3$ für Wasserstoff oder Alkyl mit 1 bis 4 C-Atomen steht,

$R^4$ für Wasserstoff oder Alkyl mit 1 bis 4 C-Atomen steht,

$R^5$ für Wasserstoff oder Alkyl mit 1 bis 4 C-Atomen steht,

$R^6$ für Furyl, Tetrahydrofuryl, Thiophenyl, Tetrahydrothiophenyl ; gegebenenfalls durch $C_1$-$C_2$-Alkyl substituiertes Isoxazolyl ; gegebenenfalls durch Cyano oder Thiocyano substituiertes $C_1$-$C_4$-Alkyl, $C_2$-$C_4$-Alkenyl und $C_2$-$C_4$-Alkinyl ; $C_1$-$C_2$-Dihalogenalkyl ; sowie die Gruppierungen $—CH_2—Az$, $—CH_2—OR^7$, $—CH_2—SR^7$, $—OR^7—SR^7$, $—CH_2—OSO_2R^7$, $—COOR^7$ und $—CH_2—O—$ 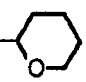

steht, wobei

$R^7$ für gegebenenfalls durch Halogen, Cyano und Thiocyano substituiertes Alkyl mit 1 bis 4 C-Atomen, $C_2$-$C_4$-Alkenyl, $C_2$-$C_4$-Alkinyl und Alkoxyalkyl mit 1 bis 4 C-Atomen steht, und

Az für Pyrazol-1-yl, 1,2,4-Triazol-1-yl und Imidazol-1-yl steht,

dadurch gekennzeichnet, daß man N-Propargyl-acetanilide der allgemeinen Formel II

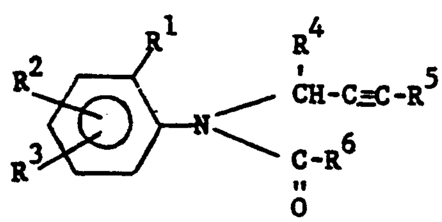

(II)

in welcher

$R^1$ bis $R^6$ die obenangegebene Bedeutung haben,

in Gegenwart einer Base als Katalysator und gegebenenfalls in Gegenwart eines Verdünnungsmittels umlagert.

3. Fungizide Mittel, gekennzeichnet durch einen Gehalt an mindestens einem N-Allenyl-acetanilid der allgemeinen Formel I.

4. Verfahren zur Bekämpfung von Pilzen, dadurch gekennzeichnet, daß man N-Allenyl-acetanilide der allgemeinen Formel I auf Pilze oder ihren Lebensraum einwirken läßt.

5. Verfahren zur Herstellung von fungiziden Mitteln, dadurch gekennzeichnet, daß man N-Allenyl-acetanilide der allgemeinen Formel I mit Streckmitteln und/oder oberflächenaktiven Mitteln vermischt.

**Claims**

1. N-Allenyl-acetanilides of the general formula I

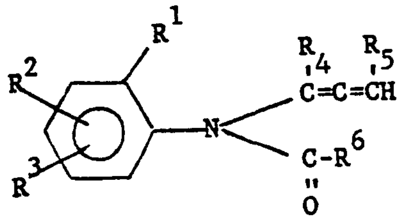

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen or alkyl with 1 to 4 carbon atoms,

$R^5$ represents hydrogen or alkyl with 1 to 4 carbon atoms,

$R^6$ represents furyl, tetrahydrofuryl, thiophenyl or tetrahydrothiophenyl ; isoxazolyl which is optionally substituted by $C_1$-$C_2$-alkyl ; $C_1$-$C_4$-alkyl, $C_2$-$C_4$-alkenyl or $C_2$-$C_4$-alkynyl, optionally substituted by cyano or thiocyano ; $C_1$-$C_2$-dihalogenoalkyl ; or the grouping $—CH_2—Az$, $—CH_2—OR^7$, $—CH_2—SR^7$, $—OR^7$, $—SR^7$, $—CH_2—OSO_2R^7$, $—COOR^7$ or $—CH_2—O—$ 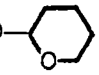 ,

wherein

$R^7$ represents alkyl with 1 to 4 carbon atoms, $C_2$-$C_4$-alkenyl, $C_2$-$C_4$-alkynyl or alkoxyalkyl with 1 to 4 carbon atoms, optionally substituted by halogen, cyano or thiocyano, and

Az represents pyrazol-1-yl, 1,2,4-triazol-1-yl or imidazol-1-yl.

2. Process for the preparation of N-allenyl-acetanilides of the general formula I

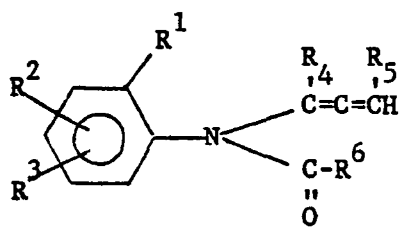

(I)

in which

$R^1$ represents hydrogen, alkyl with 1 to 4 carbon atoms or halogen,

$R^2$ represents hydrogen or alkyl with 1 to 4 carbon atoms,

$R^3$ represents hydrogen or alkyl with 1 to 4 carbon atoms,

$R^4$ represents hydrogen or alkyl with 1 to 4 carbon atoms,

$R^5$ represents hydrogen or alkyl with 1 to 4 carbon atoms,

$R^6$ represents furyl, tetrahydrofuryl, thiophenyl or tetrahydrothiophenyl ; isoxazolyl which is optionally substituted by $C_1$-$C_2$-alkyl ; $C_1$-$C_4$-alkyl, $C_2$-$C_4$-alkenyl or $C_2$-$C_4$-alkynyl, optionally substituted by cyano or thiocyano ; $C_1$-$C_2$-dihalogenoalkyl ; or the grouping $—CH_2—Az$, $—CH_2—OR^7$, $—CH_2—SR^7$, $—OR^7$, $—SR'$, $—CH_2—OSO_2R^7$, $—COOR^7$ or $—CH_2—O—$ 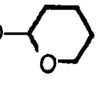 ,

wherein

$R^7$ represents alkyl with 1 to 4 carbon atoms, $C_2$-$C_4$-alkenyl, $C_2$-$C_4$-alkynyl or alkoxyalkyl with 1 to 4 carbon atoms, optionally substituted by halogen, cyano or thiocyano, and

Az represents pyrazol-1-yl, 1,2,4-triazol-1-yl or imidazol-1-yl,

characterised in that N-propargyl acetanilides of the general formula II

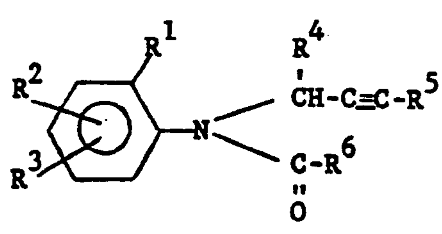

(II)

in which

$R^1$ to $R^6$ have the meaning indicated above, are subjected to a rearrangement reaction in the presence of a base, as a catalyst, and if appropriate in the presence of a diluent.

3. Fungicidal agents, characterised in that they contain at least one N-allenyl-acetanilide of the general formula I.

4. Process for combating fungi, characterised in that N-allenyl-acetanilides of the general formula I are allowed to act on fungi or their environment.

5. Process for the preparation of fungicidal agents, characterised in that N-allenyl-acetanilides of the general formula I are mixed with extenders and/or surface-active agents.

**Revendications**

1. N-allényl-acétanilides de formule générale I

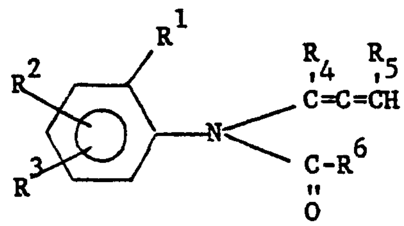

(I)

dans laquelle

$R^1$ représente l'hydrogène, un groupe alkyle en $C_1$-$C_4$ ou un halogène,

$R^2$ représente l'hydrogène ou un groupe alkyle en $C_1$-$C_4$,

$R^3$ représente l'hydrogène ou un groupe alkyle en $C_1$-$C_4$,

$R^4$ représente l'hydrogène ou un groupe alkyle en $C_1$-$C_4$,

$R^5$ représente l'hydrogène ou un groupe alkyle en $C_1$-$C_4$,

$R^6$ représente un groupe furyle, tétrahydrofuryle, thiophényle, tétrahydrothiophényle ; un groupe isoxazolyle éventuellement substitué par un groupe alkyle en $C_1$-$C_2$ ; un groupe alkyle en $C_1$-$C_4$, alcényle en $C_2$-$C_4$ éventuellement substitué par des groupes cyano ou thiocyano ; un groupe dihalogénoalkyle en $C_1$-$C_2$ ; ainsi que les groupements $—CH^2—Az$, $—CH_2OR^7$, $—CH_2—SR^7$, $—OR^7$, $—SR^7$, $—CH_2OSO_2R^7$, $—COOR^7$ et $—CH_2—O—$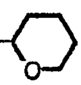 ,

$R^7$ représente un groupe alkyle en $C_1$-$C_4$, alcényle en $C_2$-$C_4$, alcynyle en $C_2$-$C_4$ ou alcoxyalkyle en $C_1$-$C_4$ éventuellement substitué par des halogènes, des groupes cyano et thiocyano, et

Az représente un groupe pyrazole-1-yle, 1,2,4-triazole-1-yle ou imidazole-1-yle.

2. Procédé de préparation des N-allényl-acétanilides de formule générale I

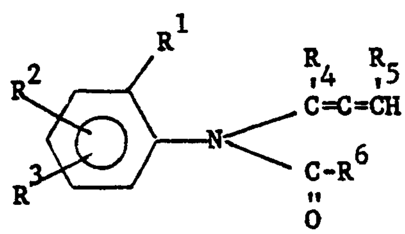

(I)

dans laquelle

$R^1$ représente l'hydrogène, un groupe alkyle en $C_1$-$C_4$ ou un halogène,

$R^2$ représente l'hydrogène ou un groupe alkyle en $C_1$-$C_4$,

$R^3$ représente l'hydrogène ou un groupe alkyle en $C_1$-$C_4$,

$R^4$ représente l'hydrogène ou un groupe alkyle en $C_1$-$C_4$,

$R^5$ représente l'hydrogène ou un groupe alkyle en $C_1$-$C_4$,

$R^6$ représente un groupe furyle, tétrahydrofuryle, thiophényle, tétrahydrothiophényle ; un groupe isoxazolyle éventuellement substitué par un groupe alkyle en $C_1$-$C_2$ ; un groupe alkyle en $C_1$-$C_4$, alcényle en $C_2$-$C_4$ et alcynyle en $C_2$-$C_4$ éventuellement substitué par un groupe cyano ou thiocyano ; un groupe dihalogénoalkyle en $C_1$-$C_2$ ; ainsi que les groupements $—CH_2—Az$, $—CH_2—OR^7$, $—CH_2—SR^7$, $—OR^7$, $—SR^7$, $—CH_2—OSO_2R^7$, $—COOR^7$ et $—CH_2—O—$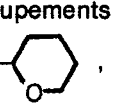 ,

$R^7$ représente un groupe alkyle en $C_1$-$C_4$, alcényle en $C_2$-$C_4$, alcynyle en $C_2$-$C_4$ et alcoxyalkyle en $C_1$-$C_4$ éventuellement substitué par des halogènes, des groupes cyano et thiocyano, et

Az représente un groupe pyrazole-1-yle, 1,2,4-triazole-1-yle et imidazole-1-yle, caractérisé en ce que l'on soumet un N-propargyl-acétanilide de formule générale II

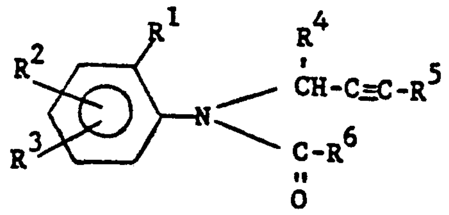

(II)

dans laquelle

$R^1$ à $R^6$ ont les significations indiquées ci-dessus, à transposition en présence d'une base servant de catalyseur et, le cas échéant, en présence d'un diluant.

3. Produits fongicides, caractérisés en ce qu'ils contiennent au moins un N-allényl-acétanilide de formule générale I.

4. Procédé pour combattre les mycètes, caractérisé en ce que l'on fait agir des N-allényl-acétanilides de formule générale I sur des mycètes ou leur habitat.

5. Procédé de préparation de produits fongicides, caractérisé en ce que l'on mélange des N-allényl-acétanilides de formule générale I avec des diluants et/ou des agents tensioactifs.